# EUROPEAN PATENT SPECIFICATION

(11) **EP 1 944 037 B1**
(45) Date of publication and mention of the grant of the patent: **29.06.2011**
(21) Application number: 06797366.9
(22) Date of filing: 04.09.2006
(51) Int. Cl.: A61K 38/21, A61P 15/14

(54) **REMEDY FOR MASTITIS**
MITTEL ZUR BEHANDLUNG VON MASTITIS
REMEDE POUR LA MASTITE

(30) Priority: 27.09.2005 JP 2005280317
(43) Date of publication of application: 16.07.2008
(73) Proprietor: KABUSHIKI KAISHA HAYASHIBARA SEIBUTSU KAGAKU KENKYUJO, Okayama-shi, Okayama 700-0907 (JP); Biovet, Inc., Tokyo 150-0002 (JP)
(72) Inventor: TAMURA, Takatoshi, Tokyo 1530044 (JP); MIWA, Yoshikatsu, Okayama-shi Okayama 7000907 (JP)
(74) Representative: Daniels, Jeffrey Nicholas
(86) International application number: PCT/JP2006/317441
(87) International publication number: WO 2007/037099

(56) References cited:
- EP-A- 0 405 315
- EP-A- 0 428 876
- EP-A- 0 537 437
- JP-A- 03 167 135
- US-A- 5 234 684
- SORDILLO L M ET AL: "Controlling acute Escherichia coli mastitis during the periparturient period with recombinant bovine interferon gamma" VETERINARY MICROBIOLOGY, ELSEVIER BV, NL, vol. 28, no. 2, 1 July 1991 (1991-07-01), pages 189-198, XP023915189 ISSN: 0378-1135 [retrieved on 1991-07-01]
- MOORE B R: "Clinical application of interferons in large animal medicine." JOURNAL OF THE AMERICAN VETERINARY MEDICAL ASSOCIATION 15 MAY 1996, vol. 208, no. 10, 15 May 1996 (1996-05-15), pages 1711-1715, XP009118888 ISSN: 0003-1488
- FOX L K ET AL: "THE EFFECT OF INTERFERON-GAMMA INTRAMAMMARY ADMINISTRATION ON MAMMARY PHAGOCYTE FUNCTION" JOURNAL OF VETERINARY MEDICINE SERIES B, vol. 37, no. 1, 1990, pages 28-30, XP009118887 ISSN: 0931-1793
- SORDILLO J.M.: 'Effect of Interferon-gamma on the Production of Tumor Necrosis Factor During Acute Escherichia coli Mastitis' JOURNAL OF DAIRY SCIENCE vol. 75, no. 8, 1992, pages 2119 - 2125, XP003012401
- BABIUK L.A.: 'Symposium: Immunobiology of Cytokines and Their Application in Disease Prevention in Dairy Cattle' JOURNAL OF DAIRY SCIENCE vol. 74, no. 12, 1991, pages 4385 - 4398, XP003012402

## Description

### Technical Field

The present invention relates to an agent for treating mastitis, more particularly, to an agent for treating mastitis, which contains interferon as an effective ingredient.

### Background Art

It is known that mammals in lactation period tend to increase frequency of suffering from mastitis as a result of microbial infection induced by various factors such as physical stimulation during lactation, occurrence of injury generated around papillae, stress inducible by changing in circumstances and delivery, and by reduction of immunity inducible thereby. In particular, during raising, mastitis are most frequently induced in dairy cows required for continuously milking well and many of which may be accidentally died and therefore it is recognized as a badly-damage-inducible disease.

Most of mastitis in dairy cows are said to be inducible by microbial infection and may be greatly influenced by environmental factors such as stress. Particularly, there has been known that the frequency of onset of mastitis is increased by heat stress frequently induced in summer and other stress induced during or after a long distance transportation, as well as immunity reduction induced by these stress. As described above, induction mechanism of mastitis is rather complicated due to the above plural factors' involvement and this makes its treatment difficult.

Mastitis includes systemic symptoms such as fever and activity reduction; clinical mastitis with apparent symptoms such as mammary disorder, milk disorder, and microbial infection; and latent mastitis, where milk disorder that accompanies the reduction of milk secretion, particularly, abnormal milk in which somatic cells are secreted, and latent mastitis where microbial infection is observed.

The above-identified mastitis is generally treated by administering antibiotics and hormones, and various other therapeutic methods have been proposed (see, for example, Japanese Patent Laid-Open Nos. 2001-213784 and 2003-171291). Since these antibiotics and hormones, however, may vary in their effects depending on infection sources and possibly induce drug resistant microbes or cause immunity reduction, and tractable mastitis could possibly be changed into intractable one. Increasing drug resistant microbes is not preferable in view of public health and actually there has not yet been established any sufficiently effective therapy for mastitis including latent mastitis. As described above, mastitis is a disease that causes economically serious damage because it is frequently found in dairy cows and may lower milk secretion or even stop milking. Mammals including humans in milk are rather susceptible to mastitis and therefore a feasible, effective prophylactic and/or therapeutic agent for mammalian mastitis has been desired.

Recently, it is reported that an oral administration of a relatively small amount of a homo- or hetero-interferon (may be abbreviated as "IFN", hereinafter) effectively treats diseases of warm-blooded animals including mammals and fowls, etc., such as dairy cows/cattle, cats/felines, pigs/swine, rats, mice, and hens/cocks (see, for example, United States Patent No. 5910304, Japanese Patent Laid-Open No. 340549/94, and *"*Clinical study and microorganisms", edited by Jeseph M. Cummins and William E. Stewart II, Vol. 18, No. 5, pp. 631-635 (1991). The same applicants as the present invention proposed that a preparation containing a small amount of human interferon-α (may be abbreviated as "HuIFN-α", hereinafter) is effective as an agent for treating cattle digestive disorders (see, for example, Japanese Patent Laid-Open No. 2005-89301). These literatures, however, neither disclose nor suggest the fact that interferons are useful as prophylactic and/or therapeutic agents for mastitis (both may be called "agents for treating mastitis", hereinafter)
SORDILLO L M ET AL: "Controlling acute Escherichia coli mastitis during the periparturient period with recombinant bovine interferon gamma" VETERINARY MICROBIOLOGY, ELSEVIER BV, NL, vol. 28, no. 2, 1 July 1991 (1991-07-01), pages 189-198, XP023915189 ISSN: 0378-1135 [retrieved on 1991-07-01] discloses the use of bovine interferon gamma for treating mastitis in cows.
US 5 234 684 discloses a method for treating or preventing mastitis in cows by administration of bovine interferon-gamma by intramammary injection.
EP 0428 876 discloses a method for treating or preventing mastitis in cows by administering interferon gamma by intramammary injection.
EP 0537 437 discloses the use of interferon for preventing or treating mastitis.
EP 0405 315 discloses the use of interferon for preventing or treating mastitis.
MOORE B R: "Clinical application of interferons in large animal medicine". JOURNAL OF THE AMERICAN VETERINARY MEDICAL ASSOCIATION 15 MAY 1996, vol. 208, no. 10, 15 May 1996 (1996-05-15), pages 1711-1715. XP009118888 ISSN: 0003-1488 discloses the use of bovine interferon gamma for the prevention of bovine mastitis.
FOX L K ET AL: "THE EFFECT OF INTERFERON-GAMMA INTRAMAMMARY ADMINISTRATION ON MAMMARY PHAGOCYTE FUNCTION" JOURNAL OF VETERINARY MEDICINE SERIES B, vol. 37, no. 1, 1990, pages 28-30, XP009118887 ISSN: 0931-1793 discloses the effect of interferon gamma on mammary phagocyte function.
SORDILLO J M: "Effect of Interferon-gamma on the Production of Tumor Necrosis Factor During Acute Escherichia coli Mastitis" JOURNAL OF DAIRY SCIENCE vol., 75, no. 8, 1992, pages 2119-2125, XP003012401 discloses the use of interferon gamma for the prevention of mastitis in cows.

### Disclosure of Invention

The present invention has a first object to provide an agent for oral use for treating mastitis in bovine, which comprises human interferon-α as an effective ingredient, wherein said human interferon-α contains interferon-α subtype α8 and interferon-α subtype α2 in an activity ratio of 1:9 to 8:2, which imposes little labor or burden to care workers and little economical load on owners, and has scarce risk in administering to animals; and has a second object to provide use of an agent for oral use as defined above for the preparation of a medicament for treating mastitis in a bovine.

To solve the above objects, the present inventors have researched on methods for preventing and treating mastitis by using IFNs for a long time. As a result, they found that HuIFNs, particularly, HuIFN-α preparations are effective on preventing and treating mastitis in mammals including humans; and among such HuIFN-α preparations, those which contain at least 10% of HuIFN subtype a8 to the total amount of HuIFN-α activity exert a distinct effect. They also found that IFNs derived from animals susceptible to mastitis exert a distinct prophylactic and therapeutic effect on such animals' mastitis, and established an agent for treating mastitis and a method for treating such disease by using the agent. Thus, they accomplished this invention. The present invention solves the above objects by providing an agent for oral use for treating mastitis in bovine, which comprises human interferon-α as an effective ingredient, wherein said human interferon-α contains interferon-α subtype α8 and interferon-α subtype α2 in an activity ratio of 1:9 to 8:2.

The agent for treating mammalian mastitis, which contains IFN(s) as an effective ingredient, and its use in the preparation of a medicament for treating mastitis according to the present invention exert a distinct effect on its prevention, improvement, and complete curing (all of which may be called as "treatment", hereinafter) of clinical symptoms of mastitis.

### Best Mode for Carrying Out the Invention

The IFN preparations usable in the present invention have a relatively high therapeutic effect on mammalian mastitis usually with only a distinctly low dose of about 0.005 to 5,000 international units (IU) per kg body weight of a mammal to be treated.

IFNs exert distinct therapeutic effects on mammalian mastitis independently of oral or parenteral administration route.

The animals to be administered with the agent of the present invention are bovine animals particularly dairy cows forced to secrete excessive amount of milk by milking. The agent of the present invention can be administered not only to the above animals for the purpose of treating mastitis but also to preclinical animals to prevent the onset of such disease.

The term "mastitis" as referred to as in the present invention means inflammation generated in mammalian mammae and it includes human mastadenitis. Explaining mastitis with reference to dairy cows which are highly susceptible to such disease at a quite high frequency of morbidity rate, the disease is directly induced as a result of intrabreast infection with microorganisms which consist mainly of bacteria existing on the body surface of dairy cows and environment where they grow, or various physicochemical factors such as stimulations by suckling. Most of mastitis in dairy cows are induced by bacteria which exist in infected milk, wounds of mamillae, wounds of human hands and foots, etc.; infectious bacteria transmittable to herds, such as *Staphylococcus aureus* and agalactous *Streptococcus;* and environmental microorganisms, usually existing in the body surface, spreading materials, and feces, such as *Staphylococcus* other than *Staphylococcus aureus,* environmental *Streptococcus,* and *Escherichia coli.* Environmental factors deeply influence on the onset of mastitis; the form of spreading materials which easily pollute mammae with feces, care for mammae during suckling, and other factors such as genetics, status, and changing of seasons and climates. In addition, mastitis may be induced by immunity reduction depending on stress, particularly, heat stress frequently found in summer, and other stress of long-distance transportation. Mastitis may be transient in some cases but most of which are hard to cure completely and which may be changed into chronic ones. Symptoms of these mastitis are systemic ones such as fever and spiritual reduction (peracute period or acute period), breast abnormality (peracute period or chronic period), milk disorder (peracute period or chronic period), clinical mastitis observed with microbial infection, and other milk disorders with no such clinical symptoms as mentioned above, particularly, latent mastitis where abnormal milk in which a number of somatic cells are secreted or microbial infection is observed. In the case of latent mastitis, reduction of milk production and secretion of abnormal milk will occur. The agent for treating mastitis of the present invention can be used in treating mastitis with the above-identified symptoms induced by the above factors. Also the agent exerts a remarkable effect on intractable mastitis which has not been effectively cured or sufficiently cured with antibiotics or hormones.

IFNs can be used in the agent for treating mammalian mastitis of the present invention independently of their origins and preparation methods as long as they have a therapeutic effect on mastitis of animals to be treated. Usually, IFNs can be used as effective ingredients after purifying in conventional manner either natural IFNs produced by cells derived from animals to be treated or recombinant IFNs produced by microorganisms, animal cells, plant cells, animals, or plants into which IFN-related-genes collected from the animal cells have been introduced. Examples of such recombinant IFNs include those which have animo acid sequences of natural IFNs; and any other IFNs as long as they have a therapeutic effect on mammalian mastitis, such as those which have an additional partial amino acids, a defective partial amino acid, or a replaced partial amino acid in the amino acid sequences of natural IFNs; and those which have a consensus sequence introduced with active parts of IFN subtypes. These IFNs can be freely used as effective ingredients in the agent for treating mastitis of the present invention. Further, chemically-modified IFNs, such as gradually degradable IFNs coupled with high molecules such as polyethylene glycol, can be used.

At present, based on their antigenicity, physiological properties, etc., IFNs are mainly classified into three kinds and two types; type I IFN (IFN-α, IFN-β (may be called IFN-α/β depending on the types of animals)), and type II IFN (IFN-γ).

These IFNs have a somewhat different therapeutic effect on mastitis. The agent of the present invention comprises human interferon-α, wherein said human interferon-α contains interferon-α subtype α8 and subtype α2 in an activity ratio of 1:9 to 8:2. Other IFN called interferon-tau can be used.

Among which, any of human IFNs produced by human cells, IFNs containing human IFNs as effective ingredients produced by microorganisms, animals, plants, or cells thereof, into which human IFN-related genes collected from human cells have been introduced, exert an improved therapeutic effect on mastitis of animals in general including cows and can be used widely and particularly advantageously used. It is known that IFN-α has subtypes and one or more of which can be arbitrarily used in combination. The agent of the present invention comprises human interferon-α, wherein said human interferon-α contains interferon-α subtype α8 and subtype α2 in an activity ratio of 1:9 to 8:2.

In use, preferably used are any of the above-identified IFNs with a highest possible level of specific activity. For oral administration, purified products with a specific activity of at least about 10⁵ international units (IU) per milligram protein; while for parenteral administration, those with a specific activity of at least about 10⁷ IU/mg protein can be preferably used. Among which, those which are free of pyrogen are particularly desirable.

The present inventors' experiments revealed that, among these HuIFNs, so called "natural HuIFN-α" produced by human lymphoblastoid cell lines such as BALL-1 cells and Namalwa cells have an improved therapeutic effect on mastitis of humans and animals including dairy cows with only a slight side effect.

The agent of the invention contains HuIFN-α with subtype α8 and HuIFN-α subtype α2 (called "subtype α2", hereinafter) in an amount with an activity ratio of 1:9 to 8:2 because of their strong therapeutic effect. Further, those which contain HuIFN-α with subtypes α8 and α2 in an amount with an activity ratio of 2:8 to 4:6 are more particularly desirable because of their distinctly strong therapeutic effect. HuIFN-α derived from BALL-1 cells, which contains subtype α8 in an amount of about 25% activity to the total HuIFN-α activity and contains subtypes α8 and α2 in a quite-well balanced activity ratio of 1:3 in terms of their specific activities, gave the most superior therapeutic effect in practicing the present invention. A combination use of HuIFN-α and human IFN-β or human IFN-γ was also confirmed that the therapeutic effect of HuIFN-α is synergistically enhanced.

The agent for treating mastitis of the present invention includes preparations in the form of IFN(s) alone or a composition containing IFN(s) and other physiologically and pharmaceutically acceptable materials such as carriers, excipients, diluents, stabilizers, gradually releasing agents, and emulsifiers; and optionally antipyretics, anti-inflamatories, antiseptics, antiviral agents, hormones, immunomodulators, digesters, nutrients, feeds, etc. The agent of the present invention can be also in the form of a medicament in a single dose unit, which means a medicament containing IFN as an effective ingredient in an amount of, for example, a daily dose, a several-fold dose of the daily dose multiplied by integers up to four, or a divisor of the daily dose up to 1/4, and which has a physically separable form suitable for objective administration. In the case of using two or more of different types of IFNs and/or different subtypes of IFN-α, single agents consisting of respective types of IFNs and/or subtypes of IFN-α can be used in combination, two or more types of IFNs can be used after mixed into a single agent, or the above agents can be arbitrarily used in combination. Examples of the above-identified agents include powders, orally administrable gel agents, granules, liquids, tablets, capsules, sublingual formulations, injections, and orally administrable film preparations. When in use, such powders and granules can be arbitrarily used after dissolving in distilled water, physiological saline, milk, substitutes for milk, etc.

Explaining the dose and use of the agent for treating mastitis of the present invention with reference to the use of HuIFN as the agent, the agent exerts the desired prophylactic and/or therapeutic effects independently of its oral or parenteral administration route. Concretely speaking, varying depending on the kinds of animals to be administered, types of causative microorganisms, symptoms, doses, uses, and the types of IFNs used, the agent is usually administered to an animal at a dose of about 0.005 to 5,000 IU/dose/kg body weight, preferably, about 0.05 to 500 IU/dose/kg body weight, and more preferably, about 0.1 to 50 IU/dose/kg body weight, and at a frequency of one to three times a day or one to six times a week for one day to six months. The present inventors' experiments revealed that the doses below the above-identified range could not attain the desired therapeutic effect, while the doses over the above-identified range may distinctly induce antibody production or side effects or may increase an economical load to an extent that cannot be ignored comparing with their therapeutic effect. Based on these, the above-identified range was determined to be the best. In the case of using IFNs derived from animals other than humans, the use and dose thereof are employed in accordance with the case of using HuIFN. In such case, suitable assays for IFNs may vary to some extent depending on their origins of animal species, however, the activity of IFNs can be assayed using systems, where IFNs inhibit cytopathic effect by viruses such as vesicular stomatitis virus (VSV) on mouse L₉₂₉ cells, rat XC cells, or bovine MDBK cells, and converting the assayed activity based on conventional IFN standard specimens derived from the same or related animals which produce the IFNs. In practicing such assay, the assayed data can be optionally converted based on HuIFN international standard specimens using cells susceptible to both testing IFNs of humans and animals. The activity of IFNs can be calculated by converting the assayed data in terms of respective conventional animal IFN standard specimens, based on the weight of IFN protein determined on enzyme immunoassay, etc.

Throughout the specification, the activity of HuIFN is expressed by assaying the level of HuIFN that inhibits the cytopathic effect by VSV on FL cells using the microliter technique, and converting the assayed data into those which are determined in terms of international units based on "Ga23-901-532", an international HuIFN standard specimen available from the World Health Organization.

In administering the agent for treating mastitis of the present invention orally or nasally, it can be prepared into products in an orally or transnasally administrable form, such as powders, touches, granules, liquids, tablets, sublingual formulations, feeds, etc.; and administered intact or optionally administered in a usual manner into oral cavity, nasal cavity, esophagus, or stomach after mixed with and dissolved in feeds, milk, substitutes for milk, etc., by using appropriate administration aids such as oral administration tools, sprays, and stomach sounds. In the case of administering parenterally, the injections according to the present invention can be administered intrasubcutaneously, subcutaneously, intramuscularly, intravascularly (i.e., venous or arterial administration), intramammary (including injection from papillae), or peritoneally. The agent for treating mastitis of the present invention can be arbitrarily applied to subjects using any of the above-identified agents and administration routes in an appropriate combination.

Comparing respective administration routes with respect to their therapeutic effects and administrability, oral administration has the merit that it can be easily applied to dairy cows/cattle and pigs/swine, as well as humans, and even in breeding and stock farms. When administering an accurate amount of IFN in a prescribed amount, it can be preferably administered intravascularly, intradermally, subcutaneously, or intramuscularly.

In addition to the prophylactic and therapeutic effects on mastitis, the agent for treating mastitis of the present invention can be also used as a prophylactic and/or therapeutic agent for IFN susceptive diseases of animals in general including dairy cows/cattle and pigs/swine; infectious diseases or loss of body weight induced by stress during transportation, etc.; and for growth promoting agents used in neonatal period, lactation period, and the following fattening phase. In such case, the use and dose of the agent can be chosen in accordance with the case as in the treatment of mastitis with the agent.

### <Experiment 1: Preparation of HuIFN-α specimen>

Based on the method disclosed in the later described Example 1, a purified specimen of HuIFN-α derived from BALL-1 cells (may be abbreviated as "BALL-1 IFN, hereinafter"), having a specific activity of 2 x 10⁸ IU/mg protein, while based on the method in Example 2, purified specimens of a recombinant subtype α8 with a specific activity of 2 x 10⁸ IU/mg protein and a recombinant subtype α2 with a specific activity of 7 x 10⁷ IU/mg protein were respectively prepared. In addition to these specimens, HuIFN-α induced by allowing Sendai virus (HVJ) to act on leukocytes isolated from human peripheral blood (may be abbreviated as "leukocyte IFN, hereinafter") was purified in accordance with the method disclosed by K. Cantell et al., in The Journal of General Virology, Vol. 39, pp. 541-543 (1978) to obtain a partially purified leukocyte IFN with a specific activity of 2 x 10⁶ IU/mg protein. The partially purified HuIFN was further purified on antibody column chromatography using an anti-HuIFN-α monoclonal antibody similarly as in the later described Example 1, and applied to gel filtration chromatography, which had been equilibrated with a phosphate buffer (pH 7.0) containing about 0.1 mg/ml of human albumin to obtain a solution of purified leukocyte IFN with a specific activity of 2 x 10⁸ IU/mg protein. The following experiments were conducted using these two types of HuIFN-α and recombinant subtypes α8 and α2.

### <Experiment 2: Influence of IFN on the number of somatic cells in milk secreted by cows>

It is known that cows just after delivery are susceptible to mastitis and have an increased number of somatic cells in milk. Based on this, the following experiment was conducted to examine the influence of IFN on the number of somatic cells in milk secreted by cows. In accordance with the method in the later described Example 5, the BALL-1 IFN specimen prepared in Experiment 1 was prepared into four types of powdered HuIFN-α preparations containing 4, 200, 2,000 or 40,000 IU/g of BALL-1 IFN using, as an excipient, "FINETOSE", a product name of anhydrous crystalline maltose commercialized by Hayashibara Shoji Inc., Okayama, Japan. Fifty Holsteins as subjects, about 600 kg body weight, 3 to 5 years of age, were randomly divided into groups consisting of 10 heads per group, administered with any of the above BALL-1 IFN preparations at a single dose of about 1.5 g/head on the 7th day before their expected delivery dates, which had been adjusted to give a dose of 0.01, 0.5, 5 or 100 IU/kg body weight/dose. The experiment was mainly conducted by a veterinarian and the number of somatic cells in milk was counted on the 1st day before nonlacting day (called "prior to nonlacting day", hereinafter), and on the 6th, 9th and 14th days after delivery. As a control group, the remaining 10 heads were orally administered with 1.5 g of the anhydrous crystalline maltose and the number of somatic cells in milk in each cow was counted similarly as in the group administered with HuIFN-α preparations. The mean values for the numbers of somatic cells in milk of the groups with HuIFN-α preparations and of the control group were calculated at respective measurement days, and the percentage (%) of increased/decreased number of somatic cells were calculated by dividing the number of somatic cells in milk on 6th, 9th, and 14th days after delivery in each group with the number of somatic cells before nonlacting day in each corresponding group, and multiplying the resulting respective numerals by 100 times. These results are in Table 1.

**[Table 1]**

| Observation items | Dose of HuIFN-α (IU/kg body weight) | Measurement day | | | |
|---|---|---|---|---|---|
| | | Before nonlacting | Sixth day after delivery | Ninth day after delivery | Fourteenth day after delivery |
| Percentage (%) of increased/decreased number of somatic cells | 0 (Control) | 100 | 146 | 150 | 171 |
| | 0.01 | 100 | 91 | 85 | 79 |
| | 0.5 | 100 | 69 | 61 | 67 |
| | 5 | 100 | 69 | 66 | 63 |
| | 100 | 100 | 67 | 61 | 58 |

As evident from Table 1, in the control group, the numbers of somatic cells in milk of the postpartum cows increased in a time dependent manner compared with those of the cows before nonlacting day, and it was observed the onset of mastitis or its aggravation accompanied by delivery and the following lactation. While in the groups administered with any of the HuIFN-α preparations at a dose of 0.01, 0.5, 5 or 100 IU/kg body weight, the numbers of somatic cells in milk of the postpartum cows decreased in a time dependent manner compared with those of the cows before nonlacting day. This result indicates that an oral administration of HuIFN-α preparation to dairy cows during nonlacting day at a dose of 0.01, 0.5, 5 or 100 IU/kg body weight/dose, prevents the onset of mastitis, treats aggravation thereof, and improves their milk quality.

### <Experiment 3: Influence of the types of IFNs on mastitis in cows>

It was revealed that IFNs are effective on the treatment of mastitis in dairy cows and on the improvement of their milk quality in Experiment 2, and the following experiment was further conducted to confirm whether there exists any differences in effects among the types of IFNs. Using any of the BALL-1 IFN preparation, leukocyte IFN preparation, recombinant subtype α8 preparation, and recombinant subtype α2 preparation, which had been prepared in Experiment 1, powdered HuIFN-α preparations containing 200 IU/g of HuIFN-α were prepared similarly as in Experiment 2 with, as an excipient, "FINETOSE", a product name of anhydrous crystalline maltose commercialized by Hayashibara Shoji Inc., Okayama, Japan. As for recombinant subtypes α8 and α2, seven types of powdered preparations, containing the recombinant subtypes α8 and α2 in an activity ratio of 10:0, 9:1, 8:2, 4:6, 2:8, 1:9, or 0:10, were prepared. One hundred dairy cows as subjects with symptoms of mastitis, 3 to 5 years of age, were randomly divided into groups consisting of 10 heads per group, orally administered with any of the above HuIFN-α preparations at a dose of about 1.5 g, which corresponds to a dose of about 0.5 IU/body weight/dose, once a day from the administration initiation day (1st day) through the following 5 days. As a control group, the remaining 10 heads were orally administered with 1.5 g of the anhydrous crystalline maltose once a day from the administration initiation day (1st day) through the following 5 days.

A veterinarian mainly conducted this experiment and evaluated the conditions of mastitis of the cows and their milk quality at the 7th day after their administration initiation days based on the criterion of four ranks in Table 2: "Inefficacious", "Slightly efficacious", "Efficacious", and "Distinctly efficacious". Further, Tables 3 and 4 show the evaluation results on the conditions of mammae and on their milk quality, respectively. The number of cows judged to be "Distinctly efficacious" and "Efficacious" in each group administered with any of the HuIFN-α preparations was divided with the total number of cows (10 heads) used in each experiment, and the resulting numeral was multiplied by 100 times to calculate respective effectiveness (%). The results are included in Tables 3 and 4.

**[Table 2]**

| Observation items | Criterion of evaluation | | | |
|---|---|---|---|---|
| | Inefficacious | Slightly efficacious | Efficacious | Distinctly efficacious |
| Conditions of mammae | Wholly expanded, increased in stiffness, or unchanged | Unchanged in number of stiffness but increased in wholly expansion | Reduction of wholly expansion and stiffness | Disappearance of wholly expansion and stiffness |
| Quality and amount of milk | Increased in solid materials and increased or unchanged in number of somatic cells | Unchanged in solid materials but decreased in number of somatic cells | Disappearance of solid materials and reduction of number of somatic cells | Disappearance of solid materials and reduction of number of somatic cells to the level below the standard level* |

| | | | | |
|---|---|---|---|---|
| *: Less than 3 x 10⁵ cells/ml | | | | |

**[Table 3]**

| Type of interferon | | Number of dairy cow (head) | | | | | |
|---|---|---|---|---|---|---|---|
| | | Distinctly efficacious | Efficacious | Slightly efficacious | Inefficacious | X | Y |
| BALL-1 IFN | | 6 | 2 | 2 | 0 | 8 | 80 |
| Leukocyte IFN | | 1 | 3 | 5 | 1 | 4 | 40 |
| Z | 10:0 | 1 | 4 | 3 | 2 | 5 | 50 |
| | 9:1 | 2 | 3 | 3 | 2 | 5 | 50 |
| | 8:2 | 3 | 3 | 3 | 1 | 6 | 60 |
| | 4:6 | 4 | 3 | 2 | 1 | 7 | 70 |
| | 2:8 | 5 | 2 | 3 | 0 | 7 | 70 |
| | 1:9 | 4 | 2 | 3 | 1 | 6 | 60 |
| | 0:10 | 2 | 2 | 3 | 3 | 4 | 40 |
| Control | | 0 | 1 | 1 | 8 | 1 | 10 |

| | | | | | | | |
|---|---|---|---|---|---|---|---|
| Note: The symbols "X", "Y" and "Z" mean "Therapeutically efficacious [(Distinctly efficacious) + (Efficacious)]", "Effectiveness (%)", and "Activity ratio of subtypes α8 and α2", respectively. | | | | | | | |

**[Table 4]**

| Type of interferon | | Number of dairy cow (head) | | | | | |
|---|---|---|---|---|---|---|---|
| | | Distinctly efficacious | Efficacious | Slightly efficacious | Inefficacious | X | Y |
| BALL-1 IFN | | 7 | 1 | 2 | 0 | 8 | 80 |
| Leukocyte IFN | | 2 | 2 | 5 | 1 | 4 | 40 |
| Z | 10:0 | 2 | 3 | 3 | 2 | 5 | 50 |
| | 9:1 | 2 | 3 | 3 | 2 | 5 | 50 |
| | 8:2 | 4 | 2 | 3 | 1 | 6 | 60 |
| | 4:6 | 5 | 2 | 2 | 1 | 7 | 70 |
| | 2:8 | 5 | 2 | 3 | 0 | 7 | 70 |
| | 1:9 | 5 | 1 | 3 | 1 | 6 | 60 |
| | 0:10 | 3 | 1 | 4 | 2 | 4 | 40 |
| | Control | 0 | 1 | 2 | 7 | 1 | 10 |

| | | | | | | | |
|---|---|---|---|---|---|---|---|
| Note: The symbols "X", "Y" and "Z" mean "Therapeutically efficacious [(Distinctly efficacious) + (Efficacious)]", "Effectiveness (%)", and "Activity ratio of subtypes α8 and α2", respectively. | | | | | | | |

As evident from Tables 3 and 4, every cow administered with any of the HuIFN-α preparations gave a distinctly improved conditions of mammae and an improved milk quality compared with those of the control group, revealing that the oral administration of HuIFN-α preparations improves mastitis in dairy cows. The improvement effect was most remarkable with an increased effectiveness of 80% when administered with the BALL-1 IFN preparation, containing subtype α8 in an amount of about 25% activity of the total HuIFN-α activity and having subtypes α8 and α2 in an activity ratio of 1:3. The group administered with either of the leukocyte IFN or the recombinant subtype α2 alone gave only an effectiveness of as low as 40%, while the group administered with the recombinant subtypes α8 and α2 in an activity ratio of 1:9 to 10:0 gave a higher effectiveness than that with the leukocyte IFN or the recombinant subtype α2 alone. Among which, a higher effectiveness was obtained in the group administered with any of the BALL-1 IFN preparation and those containing the recombinant subtypes α8 and α2 in an activity ratio of 1:9 to 8:2, and a particularly high effectiveness was marked in the group administered with any of the BALL-1 IFN preparation and those containing the recombinant subtypes α8 and α2 in an activity ratio of 2:8 to 4:6.

### <Experiment 4: Influence of the dose of IFN on mastitis in cows>

Based on the above findings, the following experiment was carried out to examine the influence of the dose of IFN on mastitis in cows using the BALL-1 IFN preparation that had marked the highest therapeutic effect on mastitis. Similarly as in Experiment 2, the BALL-1 IFN preparation, which had been prepared in Experiment 1, was processed into nine types of powdered HuIFN preparations containing 0.2, 2, 20, 40, 200, 20,000, 200,000, 2,000,000 or 20,000,000 IU/g of BALL-1 IFN using, as an excipient, anhydrous crystalline maltose. One hundred dairy cows as subjects diagnosed as mastitis, weighing about 600 kg/head, 3 to 6 years of age, were randomly divided into groups consisting of 10 heads per group, orally administered with any of the above IFN preparations at a dose of about 1.5 g, which corresponds to a dose of about 0.0005 to 50,000 IU/body weight/dose of HuIfN-α, as shown in Tables 5 and 6, once every day from the administration initiation day (1st day) through the following 5 days. As a control group, the remaining 10 heads were orally administered with 1.5 g of anhydrous crystalline maltose once a day from the administration initiation day (1st day) through the following 5 days.

A veterinarian mainly conducted this experiment and evaluated the conditions of mastitis in the cows and their milk quality at the 7th day after their administration initiation days based on the criterion of four ranks in Table 2: "Inefficacious". "Slightly efficacious", "Efficacious", and "Distinctly efficacious". Further, Tables 5 and 6 show the evaluation results on both the conditions of mammae and their milk quality, respectively. In the case that the groups were judged to be "Distinctly efficacious" and "Efficacious", they were evaluated as "Therapeutically effective". Upon this criterion, the number of cows judged to be "Distinctly efficacious" and "Efficacious" in each group administered with any of the HuIFN-α preparations was divided with the total number of cows (10 heads) used in each experiment, and the resulting numeral was multiplied by 100 times to calculate respective effectiveness (%). These results are included in Tables 5 and 6.

**[Table 5]**

| Dose of HuIFN-α (IU/kg body weight) | Number of dairy cow (head) | | | | | Y |
|---|---|---|---|---|---|---|
| | Distinctly efficacious | Efficacious | Slightly efficacious | Inefficacious | X | |
| 0 (Control) | 0 | 0 | 2 | 8 | 0 | 0 |
| 0.0005 | 0 | 0 | 3 | 7 | 0 | 0 |
| 0.005 | 0 | 2 | 3 | 5 | 2 | 20 |
| 0.05 | 3 | 4 | 1 | 2 | 7 | 70 |
| 0.1 | 3 | 5 | 1 | 1 | 8 | 80 |
| 0.5 | 3 | 5 | 2 | 0 | 8 | 80 |
| 50 | 5 | 3 | 2 | 0 | 8 | 80 |
| 500 | 4 | 3 | 3 | 0 | 7 | 70 |
| 5,000 | 4 | 2 | 4 | 0 | 6 | 60 |
| 50,000 | 3 | 3 | 4 | 0 | 6 | 60 |

| | | | | | | |
|---|---|---|---|---|---|---|
| Note: The symbols "X", "Y" and "Z" mean "Therapeutically efficacious [(Distinctly efficacious) + (Efficacious)]", "Effectiveness (%)", and "Activity ratio of subtypes α8 and α2", respectively. | | | | | | |

**[Table 6]**

| Dose of HuIFN-α (IU/kg body weight) | Number of dairy cow (head) | | | | | |
|---|---|---|---|---|---|---|
| | Distinctly efficacious | Efficacious | Slightly efficacious | Inefficacious | X | Y |
| 0 (Control) | 0 | 0 | 1 | 9 | 0 | 0 |
| 0.0005 | 0 | 0 | 5 | 5 | 0 | 0 |
| 0.005 | 0 | 3 | 3 | 4 | 3 | 30 |
| 0.05 | 2 | 5 | 2 | 1 | 7 | 70 |
| 0.1 | 3 | 5 | 1 | 1 | 8 | 80 |
| 0.5 | 4 | 4 | 2 | 0 | 8 | 80 |
| 50 | 5 | 3 | 2 | 0 | 8 | 80 |
| 500 | 3 | 4 | 3 | 0 | 7 | 70 |
| 5,000 | 2 | 4 | 4 | 0 | 6 | 60 |
| 50,000 | 3 | 3 | 3 | 1 | 6 | 60 |

| | | | | | | |
|---|---|---|---|---|---|---|
| Note: The symbols "X", "Y" and "Z" mean "Therapeutically efficacious [(Distinctly efficacious) + (Efficacious)]", "Effectiveness (%)", and "Activity ratio of subtypes α8 and α2", respectively. | | | | | | |

As evident from Tables 5 and 6, when administered with 0.0005 IU/kg body weight/dose of the HuIFN-α preparation, the cows in the test groups showed no improvement in the conditions of their mammae and milk quality similarly as those in the control group. While, those administered with 0.005 to 5,000 IU/kg body weight/dose of the HuIFN-α preparation showed a distinct improvement in the conditions of their mammae and milk quality. Such a distinct improved effect was marked "Distinctly efficacious" in both the conditions of mammae and milk quality of the cows in the test groups administered with a dose of 0.05 IU/kg body weight/dose or over of the HuIFN-α preparation, and the effect will be more improved at a dose of 0.1 IU/kg body weight/dose or over of the HuIFN-α preparation. However, when administered at a dose of 50,000 IU/kg body weight/dose of the HuIFN-α preparation, the cows in the test groups were evaluated as "Slightly efficacious" or "Inefficacious" and observed to have fever and reduction of both activity and milk amount, which were deemed to be potential side effects. The result indicates that the HuIFN-α preparation can treat mastitis at a dose of 0.005 to 5,000 IU/kg body weight/dose, and a desired therapeutic effect will be exerted at a dose of 0.05 to 5,000 IU/kg body weight/dose, preferably, at a dose of 0.05 to 500 IU/kg body weight/dose, and more preferably, at a dose of 0.1 to 50 IU/kg body weight/dose.

During the experiment, no distinct side effect, which was deemed to be induced by the administration of HuIFN-α preparation, was observed except for the administration at a dose of 50,000 IU/kg body weight/dose, supporting that the agent of the present invention quite effectively treats mastitis in cows and has a sufficient safeness. An assay for blood collected from a part of the cows on days 30 and 60 after the 5th administration day revealed that there induced no antibody production which was deemed to have had been induced by the HuIFN-α. The result indicates that the agent of the present invention is safe even when administered repeatedly to the same cow for the purpose of treating mastitis.

### <Experiment 5: Influence of IFN on intractable mastitis>

Hormone and antibiotic preparations have been used to treat mastitis, however, no sufficient therapeutic effect could have obtained in many cases and this is responsible for the onset of intractable mastitis in many cows. In view of this, the following experiment was conducted to examine the influence of IFN on such intractable mastitis. Similarly as in Experiment 2, BALL-1 IFN obtained in Experiment 1 was prepared into a powdered HuIFN-α preparation containing 200 IU/g of BALL-1 IFN by using anhydrous crystalline maltose as an excipient. Twenty Holsteins as subjects, about 600 kg body weight/head, 3 to 6 years of age, were randomly divided into two groups consisting of 10 heads per group, and one group of which was orally administered with the above HuIFN-α preparation at a dose of about 1.5 g/head, which corresponds to a dose of 0.5 IU/kg body weight/dose, once a day from the day of administration initiation (1st day) through the following 5 days. As a control, the remaining group was orally administered with 1.5 g of anhydrous crystalline maltose once a day from the day of administration initiation (1st day) through the following 5 days.

A veterinarian mainly conducted the experiment and daily evaluated the conditions of mastitis and milk quality of the cows for seven days starting from the day of administration initiation, where the evaluation was done based on the criterion with four ranks in Table 2: "Inefficacious", "Slightly efficacious", "Efficacious", and "Distinctly efficacious". The result is in Table 7. With this criterion, when judged to be "Distinctly efficacious" and "Efficacious", it is evaluated as "Therapeutically efficacious", and the number of cows, which were judged to be "Distinctly efficacious" and "Efficacious", was divided with the total number of cows (10 heads) used in each experiment, and the resulting numeral was multiplied by 100 times to calculate effectiveness (%). These results are included in Table 7.

**[Table 7]**

| Observation items | Administration of HuIFN-α preparation | Evaluation | Day after administration initiation | | | | | | |
|---|---|---|---|---|---|---|---|---|---|
| | | | 1 | 2 | 3 | 4 | 5 | 6 | 7 |
| Stiffness and exanthema in mammae | Yes | Distinctly efficacious | 0 | 0 | 1 | 2 | 2 | 3 | 5 |
| | | Efficacious | 0 | 1 | 3 | 3 | 4 | 5 | 3 |
| | | Slightly efficacious | 2 | 7 | 4 | 3 | 2 | 1 | 1 |
| | | Inefficacious | 8 | 2 | 2 | 2 | 2 | 1 | 1 |
| | | Effectiveness (%) | 0 | 10 | 40 | 50 | 60 | 80 | 80 |
| | No (Control) | Distinctly efficacious | 0 | 0 | 0 | 0 | 0 | 0 | 0 |
| | | Efficacious | 0 | 0 | 0 | 0 | 0 | | 0 |
| | | Slightly efficacious | 0 | 1 | 1 | 1 | 1 | 1 | 1 |
| | | Inefficacious | 10 | 9 | 9 | 9 | 9 | 9 | 9 |
| | | Effectiveness (%) | 0 | 0 | 0 | 0 | 0 | 0 | 0 |
| Milk quality and milk amount | Yes | Distinctly efficacious | 0 | 0 | 1 | 2 | 2 | 4 | 6 |
| | | Efficacious | 0 | 2 | 2 | 4 | 4 | 4 | 2 |
| | | Slightly efficacious | 2 | 6 | 5 | 2 | 2 | 1 | 2 |
| | | Inefficacious | 8 | 2 | 2 | 2 | 2 | 1 | 0 |
| | | Effectiveness (%) | 0 | 20 | 30 | 60 | 60 | 80 | 80 |
| | No (Control) | Distinctly efficacious | 0 | 0 | 0 | 0 | 0 | 0 | 0 |
| | | Efficacious | 0 | 0 | 0 | 0 | 0 | 0 | 0 |
| | | Slightly efficacious | 0 | 0 | 0 | 0 | 0 | 1 | 1 |
| | | Inefficacious | 10 | 10 | 10 | 10 | 10 | 9 | 9 |
| | | Effectiveness (%) | 0 | 0 | 0 | 0 | 0 | 0 | 0 |

As evident from Table 7, throughout the experimental period of time, there was no dairy cow in the control group which exhibited any improvement, marked "Distinctly efficacious" and "Efficacious" in the conditions of mammae and milk quality. While the cows orally administered with the HuIFN-α preparation which exhibited an improvement, marked "Efficacious", from the 2nd day after administration initiation day, and exhibited an improvement, marked "Distinctly efficacious", from the 3rd day after administration initiation day. On the 6th day after administration initiation day, the cows administered with the HuIFN-α preparation exhibited an effectiveness of 80% in both the conditions of mammae and milk quality.

These experimental results show that the HuIFN-α preparation is useful as an agent for treating mastitis including intractable ones in general.

The present invention is explained in detail with reference to the following Examples but they should not limit the scope of the present invention.

### Example 1

In accordance with the method disclosed in Japanese Patent Publication No. 54158/81, Sendai virus (HVJ) was allowed to act on BALL-1 cells, which had been proliferated in hamsters, in a culture medium to induce HuIFN-α. The resulting culture was centrifuged to obtain a supernatant which was then concentrated. The concentrate was applied to affinity chromatography using phenyl sepharose to obtain a partially purified HuIFN-α with a specific activity of about 10⁶ IU/mg protein. The partially purified HuIFN-α was then further purified on affinity chromatography using an antibody column, where an anti-HuIFN-α monoclonal antibody binds to a water-soluble carrier, and applied to gel filtration chromatography which had been equilibrated with phosphate buffer (pH 7.0) containing about 0.1 mg/ml of gelatin to obtain a BALL-1 IFN solution containing a purified HuIFN-α with a specific activity of about 2 x 10⁸ IU/mg protein. Comparing the activities of subtypes α8 and α2 in the solution using high-performance liquid chromatography, it was revealed that the solution contained subtypes α8 and α2 in activity percentages of about 25% and about 75% to the total activity of HuIFN-α, respectively.

The solution of a purified HuIFN-α derived from BALL-1 cells thus prepared was concentrated with a membrane up to give a concentration of about 5 mg protein per milliliter, and the concentrate was diluted with a physiological saline containing 1% (w/v) of gelatin and 0.01 M phosphate buffer (pH 7.0) to give a concentration of 300 IU/ml of HuIFN-α, and sterilized by membrane filtration. One milliliter aliquots of the resulting filtrate were aseptically injected into vials and lyophilized.

The product contains a purified HuIFN-α derived from BALL-1 cells and it is useful as an injection for treating mastitis in animals including cows.

### Example 2

### <Recombinant subtypes α2 and α8>

In accordance with the methods in Experiments 1-1(a) and 1-1(b) in Japanese Patent Laid-Open No. 2002-201141, purified specimens of recombinant subtype α8 with a specific activity of about 3 x 10⁸ IU/mg protein, and of recombinant subtype α2 with a specific activity of about 8 x 10⁷ IU/mg protein were prepared.

These specimens of subtypes α8 and α2 were respectively concentrated with a membrane up to give a concentration of about two milligrams of protein per milliliter, and mixed in an activity ratio of 1:3 (=α8:α2), and diluted with a physiological saline containing 1% (w/v) of gelatin listed in Japanese Pharmacopoeia and 0.01 M phosphate buffer (pH 7.0) to give a concentration of 100 IU/ml of HuIFN-α. The diluent was sterilized by membrane filtration, and one milliliter aliquots of the resulting filtrate were aseptically injected into vials and lyophilized.

Although the product incorporated with the purified recombinant subtypes α8 and α2 as effective ingredients is slightly inferior to the injection in Example 1 with respect to therapeutic effect and side effect, it is useful as an injection for treating mastitis of animals including cows.

### Example 3

A purified BALL-1 IFN, which had been prepared similarly as in Example 1 by allowing Sendai virus (HVJ) to act on BALL-1 cells, was diluted with a physiological saline containing 3% (w/v) of gelatin and 0.1 M phosphate buffer (pH 7.0) to give a concentration of 1,000 IU/ml of HuIFN-α. The diluent was sterilized by membrane filtration, and one milliliter aliquots of the resulting filtrate were injected into vials and lyophilized.

Similarly as the injection in Example 1, the product contains a purified HuIFN-α derived from BALL-1 cells and it is useful as an injection for treating mastitis in animals to be treated.

### Example 4

Mannitol was dissolved in distilled water to give a concentration of 2% (w/v), followed by adding to the resultant a solution, containing a partially purified HuIFN-α derived from BALL-1 cells prepared by the method in Example 1, to obtain a HuIFN-α solution containing 75 IU of HuIFN-α per one gram of the solution. Thirty-two parts by weight of the resulting solution was admixed with 0.5 part by weight of "TWEEN 80", which had been dissolved in 15 parts by weight of ethanol, and the mixture was admixed with 0.5 part by weight of triethanolamine, which had been dissolved in 15 parts by weight of glycerine, followed by adding to the resulting mixture a small amount of triethanolamine to obtain a roughly neutral gel preparation containing about 25 IU/ml of HuIFN-α.

The product, which contains HuIFN-α derived from BALL-1 cells as an effective ingredient, has an adequate oral retention time and it is an easily swallowable, oral gel preparation for treating mastitis in animals suffering from such disease.

### Example 5

One hundred parts by weight of "FINETOSE", a product name of anhydrous crystalline maltose commercialized by Hayashibara Shoji Inc., Okayama, Japan, was homogeneously sprayed with three parts by weight of a purified HuIFN-α, having a specific activity of about 10⁸ IU/ml and derived from BALL-1 cells, which had been obtained by the method in Example 1, dried *in vacuo,* pulverized, and sieved to obtain a powdered product with a particle size of 100 to 500 p. The powdered product was mixed to homogeneity with an adequate amount of "FINETOSE" to obtain a spray containing about 300 IU/g of HuIFN-α.

The product, which has an adequate oral retention time and an improved storage stability, is useful as an agent for treating mastitis in animals. Since the product has a preferable sweetness and a lesser stimulation even when administered to animals' oral cavities, it is an easily swallowable spray for animals to be treated.

### Example 6

One hundred parts by weight of "FINETOSE", a product name of anhydrous crystalline maltose commercialized by Hayashibara Shoji Inc., Okayama, Japan, was mixed to homogeneity with 15 parts by weight of a solution containing about 2,000 IU/ml of a partially purified HuIFN derived from BALL-1 cells, which had been prepared by the method in Example 1. The resulting mixture was tabletted by a tabletting machine to obtain a granule.

The product, which contains about 250 IU/g of HuIFN-α and has an improved storage stability, is useful as a powder for treating mastitis in animals. The product is an easily swallowable granule for animals to be treated because it has an adequate sweetness without substantially inducing stimulation even when administered to their oral cavities.

Five healthy cows were respectively administered with 0.8 g of the product once on the 7th day prior to expected date of delivery. While eight healthy cows were respectively administered with 0.8 g of the product once a day for successive three days from the 7th day prior to expected date of delivery. The numbers of somatic cells in milk of the cows at one and two months after delivery and calculated for their mean values, revealing that they were respectively 117,000 cells/ml and 74,000 cells/ml, while those in the cows received with three times of administration were respectively 117,000 cells/ml and 61,000 cells/ml. The mean values of the numbers of somatic cells in milk in the cows with no administration at one and two months after delivery were respectively 66,000 cells/ml and 133,000 cells/ml. In the case of being administered with the product independently of its one or three times of administration, the numbers of somatic cells in milk in the cows at two months after delivery were lower than those at one month after delivery, while the cows with no administration gave more increased numbers of somatic cells at two months after delivery than those at one month after delivery. The fact reveals that the product has an inhibitory effect on mastitis.

### Example 7

One hundred parts by weight of "FINETOSE", a product name of anhydrous crystalline maltose commercialized by Hayashibara Shoji Inc., Okayama, Japan, was homogeneously sprayed with four parts by weight of a solution which had been prepared by dissolving "IFN β injection 6,000,000 I.U.", a product name of a commercialized HuIFN-β preparation containing about 100 IU/g of HuIFN-β, commercialized by Mochida Pharmaceutical Co., Ltd., Tokyo, Japan, in one milliliter of distilled water, and the resulting mixture was dried *in vacuo,* pulverized, and sieved to obtain a powdered product with a particle size of 100 to 500 p. The powdered product was mixed to homogeneity with an adequate amount of "FINETOSE" to obtain a powder containing about 100 IU/g of HuIFN-β.

Using the HuIFN-β preparation powder and 30 pigs, which were estimated to be 3 to 4 years of age and exhibited the symptoms of mastitis, the pigs were orally administered with 10 I.U./kg body weight of the HuIFN-β preparation for seven days, resulting in exhibiting an efficaciousness of about 47%. The product was judged to have a therapeutic effect on mastitis because of its significantly high efficaciousness compared with that for the group with no administration, consisting of 20 pigs and having an efficaciousness of 5%.

In accordance with the method in Experiment 3, using 20 pigs, which were estimated to be 3 to 4 years of age and exhibited the symptoms of mastitis, the pigs were orally administered for seven days with 5 I.U./kg body weight of the HuIFN-β preparation and 10 IU/kg body weight of the 200 IU/g of the recombinant subtype α8 preparation, which had been prepared in Experiment 4, resulting in exhibiting an efficaciousness of 50%. A combination use of the product and the recombinant subtype α8 preparation was judged to have a therapeutic effect on mastitis in pigs because of its significantly high efficaciousness compared with the efficaciousness of 40% for the group, consisting of 20 pigs administered with 15 IU/kg body weight of the recombinant subtype α8 preparation, and the efficaciousness of 10% for the group, consisting of 10 pigs with no administration.

The product has an adequate oral retention time and an improved storage stability. The above result also revealed that HuIFN-β preparation is useful as an agent for treating mastitis in pigs and, when used in combination with a HuIFN-α preparation, the HuIFN-β preparation augments the therapeutic effect of the HuIFN-α preparation. The product is an easily swallowable powder for animals to be treated because it has an adequate sweetness and a lesser stimulation even when administered to their oral cavities.

### Example 8

One hundred parts by weight of "FINETOSE", a product name of anhydrous crystalline maltose commercialized by Hayashibara Shoji Inc., Okayama, Japan, was homogeneously sprayed with two parts by weight of a solution which had been prepared by adding one milliliter of physiologically saline to a commercialized "INTERCAT", a product name of a lyophilized recombinant feline IFN preparation produced by Toray Industries Inc., Tokyo, Japan, into a solution with an activity of about 10⁷ units/ml. The resulting mixture was dried *in vacuo,* pulverized, and sieved into a powdered product with a particle size of 100 to 500 p. The powdered product was mixed to homogeneity with an adequate amount of "FINETOSE" to obtain a powder containing about 20 units/g of the feline IFN. The activity was calculated based on the activity described in the appendix of the "INTERCAT".

The product has an adequate oral retention time and an improved storage stability and it is useful as an agent for treating mastitis in felines. The product is an easily swallowable powder for animals to be treated because it has an adequate sweetness and a lesser stimulation even when administered to their oral cavities.

### Example 9

In accordance with the method for preparing human leukocyte interferon in Experiment 1, leukocytes which had been separated from peripheral blood of a cow were subjected to the action of Sendai virus (HVJ) to induce a bovine IFN-α, followed by purifying the IFN-α to obtain a specimen with a specific activity of 1 x 10⁷ IU/mg protein. Two parts by weight of a physiological saline of the specimen with an activity of about 10⁸ IU/ml was homogeneously sprayed over 100 parts by weight of "FINETOSE", a product name of anhydrous crystalline maltose commercialized by Hayashibara Shoji Inc., Okayama, Japan, and the mixture was dried *in vacuo,* pulverized, and sieved to obtain a powdered product with a particle size of 100 to 500 µ. The powdered product was further mixed to homogeneity with an adequate amount of "FINETOSE" to obtain a powder containing about 200 IU/g of the bovine IFN-α. The activity of bovine IFN-α of the product was determined by using MTT assay to measure the inhibitory level of the IFN-α against the cytopathicity of MDBK cells, derived from bovine kidney, by Sindbis virus, and converting the measured inhibitory level into a value of "international unit" determined based on an international standard of HuIFN-α.

The product has an adequate oral retention time and an improved storage stability and it is useful as an agent for treating mastitis in animals including cows. The product is an easily swallowable powder for animals because it has an adequate sweetness and a lesser stimulation even when administered to their oral cavities.

### Industrial Applicability

The IFNs incorporated as effective ingredients in the agent for treating mastitis of the present invention exert a distinct effect on mastitis in animals with only a small amount thereof and such a low dose administration will largely reduce the economical load on their owners. For the same reason, without necessarily requiring intravascular administration, the agent of the present invention will exert a desired effect even when administered orally or parenterally such as intradermally, subcutaneously, and intramuscularly other than intravascular administration route. Because of this, a prescribed amount of HuIFN can be surely and easily administered even to nervous, highly-cautious animals, and this will reduce labor/burden on care workers such as doctors and veterinarians. The agent of the present invention exerts a distinct therapeutic effect on mastitis including intractable one in dairy cows and it will highly reduce economical loss induced by mastitis. The present invention with such outstanding effects is a significant invention that will greatly contribute to the medical and livestock fields.

## Claims

1. An agent for treating mastitis in bovine via oral administration, which comprises human interferon-α as an effective ingredient, wherein said human interferon-α is administered at a dose of about 0.1 to 50 international units (IU) of interferon per kg of body weight of said bovine, and contains interferon-α subtype α8 and subtype α2 in an activity ratio of 2:8 to 4:6

2. An agent for use as defined in claim 1 for the preparation of a medicament for treating mastitis in bovine via oral administration.

## Patentansprüche

1. Mittel zur Behandlung von Rindermastitis über orale Verabreichung, welches menschliches Interferon-α als effektiven Bestandteil umfasst, wobei das Interferon-α mit einer Dosis von ca. 0.1 bis 50 internationalen Einheiten (IU) Interferon pro kg Körpergewicht des betreffenden Rindes verabreicht wird, und Interferon-α Untergruppe α8 und Untergruppe α3 in einem Aktivitätsverhältnis von 2:8 bis 4:6 enthält.

2. Mittel für eine Verwendung nach Anspruch 1 für die Herstellung eines Medikaments zur Behandlung von Rindermastitis über orale Verabreichung.

## Revendications

1. Agent pour le traitement de la mastite chez les bovins, par administration orale, qui comprend de l'interféron-α humain comme ingrédient efficace, dans lequel ledit interféron-α humain est administré à une dose d'environ 0,1 à 50 unités internationales (UI) d'interféron par kg de poids corporel dudit bovin, et contient le sous-type α8 et le sous-type α2 d'interféron-α en un rapport d'activité de 2:8 à 4:6.

2. Agent destiné à être utilisé de la manière définie dans la revendication 1, pour la préparation d'un médicament destiné au traitement de la mastite chez les bovins, par administration orale.
